(19) Europäisches Patentamt
European Patent Office
Office européen des brevets

(11) Veröffentlichungsnummer : **0 098 433**
**B1**

(12) # EUROPÄISCHE PATENTSCHRIFT

(45) Veröffentlichungstag der Patentschrift : **02.12.87**

(21) Anmeldenummer : **83105959.7**

(22) Anmeldetag : **18.06.83**

(51) Int. Cl.⁴ : **C 07 D501/20**, C 07 D499/52, C 07 D277/42, A 61 K 31/545, A 61 K 31/43// C07D277/46

(54) **Beta-Lactamantibiotika, Verfahren zu deren Herstellung sowie sie enthaltende Mittel.**

(30) Priorität : **02.07.82 DE 3224866**

(43) Veröffentlichungstag der Anmeldung : **18.01.84 Patentblatt 84/03**

(45) Bekanntmachung des Hinweises auf die Patenterteilung : **02.12.87 Patentblatt 87/49**

(84) Benannte Vertragsstaaten : **AT BE CH DE FR GB IT LI NL SE**

(56) Entgegenhaltungen :
**EP-A- 0 049 448**
**GB-A- 2 076 801**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Boberg, Michael, Dr.**
**Untere Bergerheide 47**
**D-5600 Wuppertal 1 (DE)**
Erfinder : **Metzger, Karl Georg, Dr.**
**Pahlkestrasse 15**
**D-5600 Wuppertal 1 (DE)**
Erfinder : **Zeiler, Hans-Joachim, Dr.**
**Elsbeekerstrasse 46**
**D-5620 Velbert 15 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 098 433 B1

**Beschreibung**

Die Erfindung betrifft β-Lactamverbindungen, Verfahren zu ihrer Herstellung sowie Mittel, wie Arzneimittel, insbesondere antibakterielle Mittel, ferner Mittel zur Förderung des Wachstums und zur Verbesserung der Futterverwertung bei Tieren sowie Antioxidantien.

Es sind bereits β-Lactamverbindungen, die eine substituierte Acrylamidoseitenkette tragen, beschrieden worden.

So sind z. B. in der BE-PS 633 397 und in der US-PS 3 622 569 Penicillinverbindungen erwähnt, die das Strukturelement

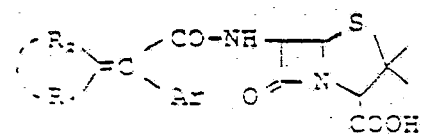

enthalten, worin Ar ein substituierter Phenylring oder ein Heterocyclus sein kann und $R^1$ und $R^2$ substituierte oder unsubstituierte Alkylgruppen oder eine Cycloalkylgruppe darstellen.

Aus der EP-A-49 448 sind antibiotisch aktive Verbindungen bekannt, die Aminothiazolyl in der Seitenkette enthalten. Des weiteren werden in der GB-2 076 801 Verbindungen des aus der EP-A-49 448 bekannten Strukturtyps beschrieben, die eine Gruppe —S— an Stelle der $—S(O)_{1,2}$-Gruppe aufweisen.

Weiterhin beschreibt die US-PS 4 014 869 Cefalosporinverbindungen mit einer Z-konfigurierten Acrylamidoseitenkette, die in 2- und 3-Stellung u. a. aromatische bzw. heterocyclische Reste trägt,

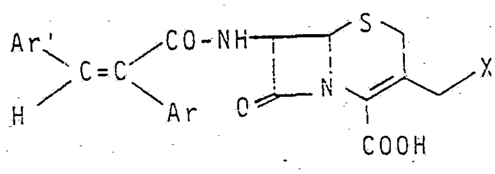

und die BE-PS 888 389 Cefalosporinverbindungen mit einer Acrylamino-Seitenkette mit aromatischen bzw. heterocyclischen 2-Substituenten und Alkylthio- bzw. Alkoxy-3-Substituenten.

Die vorliegende Erfindung betrifft β-Lactamverbindungen der allgemeinen Formel (I)

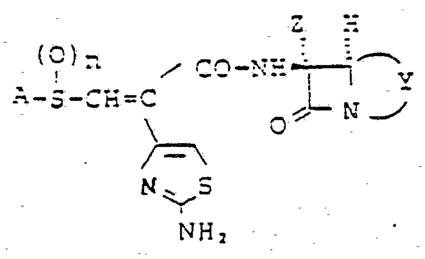

(I)

in der

Z Wasserstoff oder $C_1$-$C_6$-Alkoxy,

n 1 oder 2

Y in der Form der freien Säure einen Rest der Formel IIa oder IIb

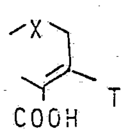

(IIa)

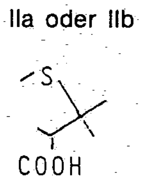

(IIb)

bedeutet, worin

X —S—, —SO— oder $—SO_2—$ und

T ein in der Cephalosporinchemie üblicher Rest ist,

und worin

A Hydroxy, Alkyl mit bis zu 18 C-Atomen, das gegebenenfalls durch Halogen, Hydroxy, $C_1$-$C_4$-Alkoxy, $OCOR^2$, Thio, eine Gruppe

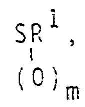

eine Gruppe

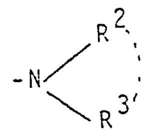

Nitro, Cyano, Hydroxycarbonyl, $C_1$-$C_4$-Alkoxycarbonyl, Aminocarbonyloxy, Sulfo oder einen 5- oder 6-gliedrigen Ring substituiert ist, wobei

$R^1$ verzweigtes oder unverzweigtes $C_1$-$C_4$-Alkyl bedeutet,

m 0, 1 oder 2 sein kann und

$R^2$ und $R^3$ gleich oder verschieden sind und Wasserstoff, $C_1$-$C_5$-Alkyl oder $C_1$-$C_5$-Alkanoyl sein können, einen Rest der Formel

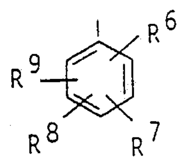

worin $R^6$, $R^7$, $R^8$ und $R^9$ unabhängig voneinander Wasserstoff, Halogen, Alkyl mit bis zu 6 Kohlenstoffatomen, Aryl, eine Gruppe —$OCOR^{10}$, eine Gruppe

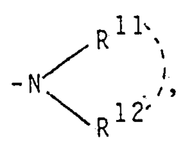

Nitro, Cyano, ($C_1$-$C_4$)-Alkoxy, ($C_1$-$C_4$)-Alkylthio, Hydroxycarbonyl, ($C_1$-$C_4$)-Alkoxycarbonyl, Aminocarbonyloxy, Sulfonyl oder Sulfo bedeutet, worin wiederum Aryl einen 5- oder 6-gliedrigen heterocyclischen Ring bedeutet, und worin $R^{10}$ verzweigtes oder unverzweigtes Alkyl mit bis zu 6 Kohlenstoffatomen bedeutet, und worin $R^{11}$ und $R^{12}$ gleich oder verschieden sind und Wasserstoff, Alkyl oder Alkanoyl sein können, jeweils mit bis zu 6 C-Atomen im Alkylteil, oder einem heterocyclischen 5-oder 6-Ring mit Stickstoff, Sauerstoff oder Schwefel als Heteroatomen im Ring bedeutet, sowie ihre nicht-toxischen pharmazeutisch verträglichen Salze.

Die Erfindung betrifft ferner Verbindungen, die der allgemeinen Formel III

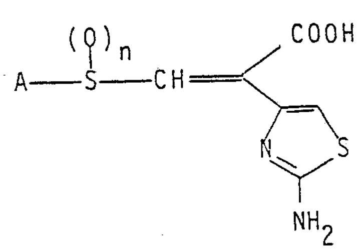

(III)

entsprechen, worin A und n die oben angegebene Bedeutung besitzen.

Die erfindungsgemäßen Verbindungen der Formel (I) haben sehr gute antibakterielle Eigenschaften, die erfindungsgemäßen Verbindungen der Formel (III) dienen als Ausgangsmaterialien zur Herstellung der Verbindungen der Formel (I).

In den Verbindungen der Formeln (I) und (III) existieren zu jeder Strukturformel eine E- und eine Z-konfigurierte Verbindung gemäß der in J. Amer. Chem. Soc. 90, 509 (1968) beschriebenen E/Z-Nomenklatur.

Bevorzugte Verbindungen der Formeln (I) und (III) liegen in der Z-Konfiguration vor.

Die Verbindungen der Formel (I) können als freie Säuren, Ester, als innere Salze oder als nicht toxische, pharmazeutische verträgliche Salze der sauren Carboxylgruppen, wie die Natrium- Kalium-, Magnesium-, Calcium-, Aluminium- und Ammoniumsalze und nicht-toxische substituierte Ammoniumsalze, mit Aminen wie Di- und Triniedrigalkylaminen, Procain, Dibenzylamin, N,N'-Dibenzylethylendiamin, N-Benzyl-β-phenylethylamin, N-Methyl- und N-Ethylmorpholin, 1-Ephenamin, Dehydroabietylamin, N,N'-Bis-dehydroabietylethylendiamin, N-Niedrigalkylpiperidin und andere Amine, die zur Bildung von Salzen von Penicillinen oder Cephalosporinen verwendet werden können, vorliegen.

Wenn A einen Alkylrest darstellt, dann bevorzugt einen geradkettigen oder verzweigten, gegebenenfalls substituierten Rest mit bis zu 18 C-Atomen, besonders bevorzugt mit bis zu 12 C-Atomen und speziell mit bis zu 6 C-Atomen.

Substituenten für Alkyl sind bevorzugt Halogen vzw. Cl, Hydroxy, Niedrigalkoxy ($C_1$-$C_4$), eine Gruppe $OCOR^2$, Thio, eine Gruppe

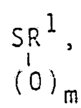

eine Gruppe

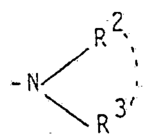

Nitro, Cyano, Hydroxycarbonyl, $C_1$-$C_4$-alkoxycarbonyl, Aminocarbonyloxy, Sulfo oder 5- oder 6-gliedrige heterocyclische Ringe, worin $R^1$ verzweigtes oder unverzweigtes $C_1$-$C_4$-Alkyl bedeutet, worin m 0, 1 oder 2 sein kann, und worin $R^2$ und $R^3$ gleich oder verschieden sind und Wasserstoff, $C_1$-$C_5$-Alkyl oder $C_1$-$C_5$-Alkanoyl sein können.

Die Bedeutung Alkyl betrifft bevorzugt Reste mit 1 bis 6 C-Atomen.

Wenn A einen Phenylrest darstellt, dann bevorzugt einen Rest der Formel

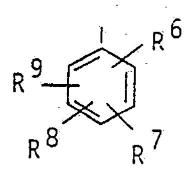

worin $R^6$, $R^7$, $R^8$ und $R^9$ unabhängig voneinander Wasserstoff, Halogen, vorzugsweise Cl, Alkyl mit bis zu 6 Kohlenstoffatomen, Aryl, eine Gruppe —$OCOR^{10}$, eine Gruppe

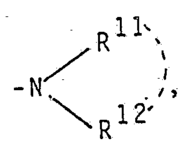

Nitro, Cyano, Niedrig-($C_1$-$C_4$)-alkoxy, ($C_1$-$C_4$)-Alkylthio, Hydroxycarbonyl, ($C_1$-$C_4$)-Alkoxycarbonyl, Aminocarbonyloxy, Sulfonyl oder Sulfo bedeutet, worin wiederum Aryl einen 5- oder 6-gliedrigen heterocyclischen Ring bedeutet und worin $R^{10}$ verzweigtes oder unverzweigtes Alkyl mit bis zu 6 Kohlenstoffatomen bedeutet und worin $R^{11}$ und $R^{12}$ gleich oder verschieden sind und Wasserstoff, Alkyl oder Alkanoyl sein können, jeweils mit bis zu 6, vorzugsweise 4 C-Atomen im Alkylteil.

Wenn Y einen Rest der Formel IIa bedeutet, dann sind bevorzugte Verbindungen der Formel I solche, in denen

Z Wasserstoff oder Methoxy,

X Schwefel oder Sauerstoff bedeutet und

T Wasserstoff, $C_1$-$C_4$-Alkyl, Halogen, $C_1$-$C_4$-Alkoxy, Hydroxymethyl, Formyloxymethyl, $C_1$-$C_4$-Alkylcarbonyloxymethyl, Aminocarbonyloxymethyl, Pyridiniummethyl, 4-Carbamoylpyridiniummethyl, Halogenmethyl vzw. Chlormethyl, Dichloracetoxymethyl, Sulfonyloxymethyl oder Heterocyclylthiomethyl bedeutet, wobei Heterocyclyl vorzugsweise für einen Rest der Formel

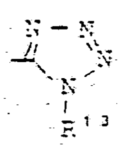 , 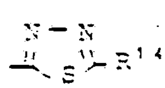 , 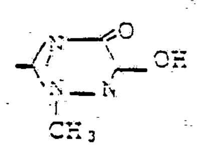 , oder

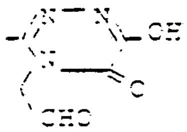

steht, worin

$R^{13}$ Wasserstoff, Methyl, 2-Dimethylaminoethyl, Sulfoaminoethyl, Carboxymethyl oder Sulfomethyl und

$R^{14}$ Wasserstoff oder Methyl bedeuten.

Besonders bevorzugte Verbindungen in der Z-Konfiguration sind in der Formel I solche, in denen

Y die Bedeutung II b oder II a hat, worin

Z Wasserstoff oder Methoxy,

X Schwefel

T Wasserstoff, Halogen oder einen Rest

$-CH_2OCOCH_3$ , 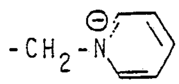 , 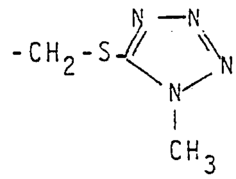 ,

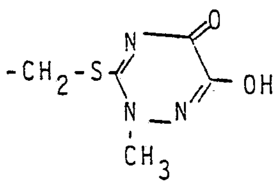 , 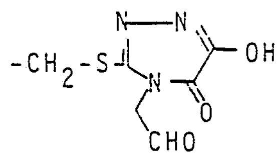 oder

$—CH_2OCONH_2$ bedeuten, und in denen

n 2 ist, und

A Hydroxy, Alkyl mit bis zu 6 Kohlenstoffatomen oder Aryl bedeutet, worin Aryl einen unsubstituierten oder substituierten Phenylrest bedeutet, worin wiederum als Substituenten bevorzugt Halogen, Alkyl mit bis zu 6 Kohlenstoffatomen, Niedrigalkoxy oder Hydroxycarbonyl stehen.

Die jeweiligen Z- und E-Formen derselben Strukturformel sind in der Regel unterschiedlich wirksame Substanzen, die getrennt voneinander oder gemeinsam hergestellt werden können.

Die Verbindungen der allgemeinen Formel I können dadurch erhalten werden, daß Verbindungen der allgemeinen Formel III

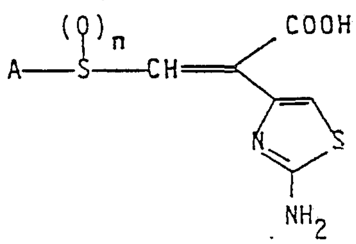

(III)

worin A und n die oben angegebene Bedeutung haben, in denen die Aminogruppe geschützt oder ungeschützt vorliegen kann, nach Aktivierung der Carboxylgruppe durch Überführung in ein gemischtes Anhydrid, beispielsweise mit Chlorameisensäureethylester oder Methansulfonsäurechlorid, nach Überführung in das Säurehalogenid oder nach Überführung in einen aktivierten Ester mit beispielsweise N-Hydroxisuccinimid und Dicyclohexylcarbodiimid, mit Verbindungen der allgemeinen Formel IV, die auch in der Mono- oder Disilylform vorliegen können und in denen die Carboxylgruppe geschützt oder ungeschützt vorliegen kann,

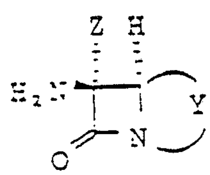

(IV)

worin Y und Z die obengenannte Bedeutung besitzen, zur Umsetzung gebracht werden, dann

gegebenenfalls Schutzgruppen abgespalten werden und die gewünschten Salze oder aus Salzen die freien Säure hergestellt werden.

Die erfindungsgemäßen Verbindungen der Formel (I) mit Y = IIa lassen sich auch erhalten durch Umsetzung von Verbindungen der Formel (I) und Y = IIa, in denen T die Bedeutung $—CH_2—O—CO-$Niedrigalkyl, insbesondere $—CH_2—OCOCH_3$, oder Halogenmethyl, Sulfonyloxymethyl oder Dichloracetoxymethyl hat, mit Nukleophilen, beispielsweise

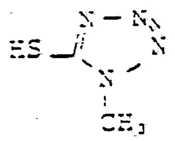 oder 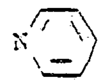

Verbindungen der Formel (I) erhält man weiterhin, wenn man Verbindungen der allgemeinen Formel (I) und Y = IIa in denen T die Bedeutung von $CH_2OH$ hat, mit $O{=}C{=}NSO_2Cl$ oder $O{=}C{=}N—COCCl_3$ umsetzt und anschließend die $SO_2Cl$- oder $—COCCl_3$-Gruppe abspaltet.

Verbindungen der Formel (I), in denen Z Alkoxy bedeutet, kann man auch erhalten, wenn man Verbindungen der Formel (I), in denen Z Wasserstoff bedeutet und die in der Form der freien Säuren, der Salze oder spaltbarer Ester vorliegen, in Gegenwart eines Alkoholats, vorzugsweise Methanolats, beispielsweise $LiOCH_3$, mit einem Hypochlorit, beispielsweise $(CH_3)_3COCl$ umsetzt und gegebenenfalls Schutzgruppen entfernt.

Die Verbindungen der allgemeinen Formel (III) können dadurch erhalten werden, daß Verbindungen der allgemeinen Formel V

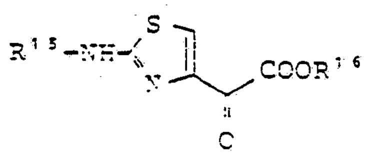

(V)

worin $R^{15}$ eine Amin-Schutzgruppe wie beispielsweise tert.-Butoxycarbonyl, Triphenylmethyl, Formyl oder 2-Trimethylsilylethoxycarbonyl bedeutet und worin $R^{16}$ gegebenenfalls substituiertes, verzweigtes oder unverzweigtes Niedrigalkyl bedeutet, mit einem Phosphonat der allgemeinen Formel (VI)

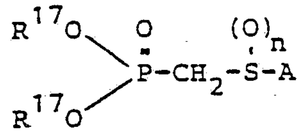

(VI)

worin A und n die obengenannte Bedeutung haben und $R^{17}$ Alkyl mit bis zu 6 C-Atomen oder Aryl, vorzugsweise Methyl Ethyl, Isopropyl oder Phenyl bedeutet, umsetzt und dann den Ester $COOR^{16}$ spaltet und gegebenenfalls auch die Schutzgruppe $R^{15}$ abspaltet.

Bevorzugte Verbindungen der Formel (V) sind solche, in denen

$R^{15}$ tert.-Butoxycarbonyl oder 2-Trimethylsilylethoxycarbonyl und

$R^{16}$ Methyl, Ethyl, tert.-Butyl, 2-Trimethylsilylethyl oder Diphenylmethyl bedeutet.

Die erfindungsgemäßen Verbindungen zeigen ein breites antibakterielles Spektrum gegen grampositive und gram-negative Keime, insbesondere gegen Enterobakteriaceae, vor allem gegen solche mit β-Lactamasebildung.

Ferner verbessern die erfindungsgemäßen Verbindungen das Wachstum und die Futterausnutzung bei Tieren und sind als Antioxidantien verwendbar.

Die erfindungsgemäßen Verbindungen weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert

werden, die durch die folgenden Erreger oder durch Mischungen der folgenden Erreger verursacht werden :

Micrococcaceae, wie Staphylokokken, z. B. Staphylococcus aureus, Staph. epidermidis, (Staph. = Staphylococcus) ; Lactobacteriaceae, wie Streptokokken, z. B. Streptococcus pyogenes, α- bzw. β-hämolysierende Streptokokken, nicht (γ-)-hämolysierende Streptokokken, Enterokokken und Dipolococcus pneumoniae (Pneumokollen) (Str.=Streptococcus) ; Enterobacteriaceae, wie Escherichiae-Bakterien der Coli-Gruppe : Escherichia-Bakterien, z. B. Escherichia coli, Enterobacter-Bakterien, z. B. E. aerogenes, E. cloacae, Klebsiella-Bakterien, z. B. K. pneumoniae, Serratia, z. B. Serratia marcescens (E. = Enterobacter) (K. = Klebsiella), Proteae-Bakterien der Proteus-Gruppe : Proteus, z. B. Proteus vulgaris, Pr. morganii, Pr. rettgeri, Pr. mirabilis, (Pr. = Proteus) ;

Pseudomonadaceae, wie Pseudomonas-Bakterien, z. B. Pseudomonas aeruginosa, (PS. = Pseudomonas) ;

Bacteroidaceae, wie Bacteroides-Bakterien, z. B. Bacteroides fragilis, (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt :

Erkrankungen der Atmungswege und des Rachenraumes ; Otitis ; Pharyngitis ; Pneumonie ; Peritonitis ; Pyelonephritis ; Cystitis ; Endocarditis ; Systeminfektionen ; Bronchitis ; Arthritis ; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, sie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quaternäre Ammoniumverbindungen, (g) Netzmittel, z. B. Cetylakohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans

oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis etwa 1 000, vorzugsweise 10 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative und grampositive Bakterien verhindert, gebessert und/oder geheilt werden werden und ebenso eine Förderung des Wachstums und eine Verbesserung der Verwertung des Futters erreicht werden.

Die neuen Verbindungen zeichnen sich durch starke antibakterielle Wirkungen, die in vivo und in vitro geprüft wurden, und durch orale Resorbierbarkeit aus.

Die erfindungsgemäßen Verbindungen können zum Zwecke der Erweiterung des Wirkungsspektrums und um eine Wirkungssteigerung speziell bei β-lactamasebildenden Bakterien zu erzielen mit anderen antimikrobiellen Wirkstoffen z. B. mit Penicillinen oder anderen Betalactamen, die besonders penicillinasefest sind, kombiniert werden. Eine solche Kombination wäre z. B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z. B. Gentamicin, Sisomicin, Kanamicin, Amikacin oder Tobramicin kombiniert werden.

Die erfindungsgemäßen Verbindungen verhindern den Abbau von z. B. Penicillinen durch β-Lactamasen, die normalerweise empfindliche Penicilline hydrolysieren und damit unwirksam machen.

Die Wirksamkeit der erfindungsgemäßen β-Lactamantibiotica kann durch die folgenden in vitro-Versuche beispielhaft demonstriert werden :

In Vitro-Versuche

Die Verbindungen der Beispiele 5 und 10, welche als typische Vertreter der erfindungsgemäßen Verbindungen betrachtet werden können, wurden im Agarverdünnungstest unter deutschen DIN-Norm-Bedingungen auf antibakterielle Wirkung geprüft. Die Konzentration betrug 100 mcg pro Milliliter Agar. Bei folgenden Bakterienstämmen wurde völlige Wachstumshemmung festgestellt :

E. coli Neumann, Klebsiella 63, Serratia marcescens 16001, Providencia 12012, Proteus morganii 11006, Proteus vulgaris 1017, Proteus rettgeri, Pseudomonas aeruginosa W, Bacteroides fragilis 012 999, Staphylococcus aureus 133, Enterococcus ATCC 9790.

Beispiel 1

2-(2-tert.-Butoxycarbonylaminothiazol-4-yl)-3-methyl-sulfonylpropensäureethylester

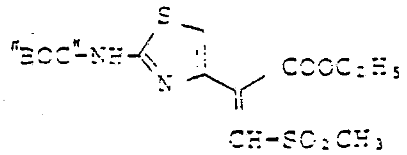

«BOC» = tert.-Butoxycarbonyl

In einem ausgeheizten Kolben werden unter Stickstoffstrom zu einer Lösung von 23 g Diethylmethylsulfonyl-methylphosphonat in 500 ml abs. THF bei — 78 °C unter Rühren 71 ml einer 1,5 normalen Lösung von N-Butyllithium in Hexan zugetropft. Anschließend tropft man eine Lösung von 30 g 2-(2-tert.-Butoxycarbonylaminothiazol-4-yl)-2-oxo-essigsäureethylester in 300 ml abs. THF ein und rührt 4 Stunden nach. Man läßt die Lösung langsam auf Raumtemperatur kommen, gießt dann auf Eis/Wasser, stellt pH 6 ein und zieht das THF ab. Die wäßrige Phase wird dreimal mit Essigester extrahiert, die vereinigten organischen Phasen werden getrocknet und eingedampft, Roh-Ausbeute 46,0 g. Chromatographie an der 80-fachen Menge Kieselgel (Laufmittel Toluol/Essigester : 4/1) liefert 9 g eines 1 : 1-Gemisches aus Z-Isomerem (unpolares Produkt) und Ausgangsmaterial, 17,2 g des reinen Z-Isomeren und 1,6 g des reinen E-Isomeren (polares Produkt). Ausbeute : 53,2 %.

Z-Isomeres

IR (Methylenchlorid) : 1 715, 1 590, 1 545, 1 440, 1 370, 1 350, 1 310, 1 225, 1 200, 1 140 $cm^{-1}$.
NMR ($CDCl_3$) : δ = 7,12(1)s, 7,09(1)s, 4,42(1)g, J = 7 Hz, 3,08(3)s, 1,57(9)s, 1,41(3)t, J = 7 Hz ppm.

E-Isomeres

IR ($CH_2Cl_2$) : 1 715, 1 540, 1 440, 1 420, 1 365, 1 310, 1 230, 1 145 $cm^{-1}$
NMR ($CDCl_3$) : δ = 7,42(1)s, 7,21(1)s, 4,26(2)q, J = 7 Hz, 3,04(3)s, 1,52(9)s, 1,31(3)t, J = 7Hz ppm.

## Beispiel 2

2-(2-tert.-Butoxycarbonylaminothiazol-4-yl)-3-methylsulfonylpropensäure-(2-trimethylsilylethyl) ester

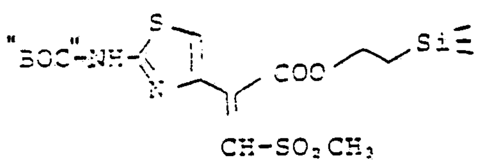

In einem ausgeheizten Kolben unter Stickstoff werden zu einer Lösung von 8,64 ml Diisopropylamin in 120 ml abs. THF bei — 70° 55,94 ml einer 1,5 m Lösung von n-Butyllithium in Hexan getropft. Nach 15 Minuten werden 17,3 g Diethylmethylsulfonylmethylphosphonat zugesetzt, nach weiteren 15 Minuten tropft man eine Lösung von 30 g 2-(2-tert.-Butoxycarbonylaminothiazol-4-yl)-2-oxoessigsäure-2-(trimethylsilyl) ethylester in 50 ml abs. THF ein. Die Reaktionsmischung wird eine Stunde bei — 40° gehalten, dann auf Raumtemperatur aufgetaut und über Nacht nachgerührt. Aufarbeitung wie in Beispiel 1 ergibt 40,3 g Rohprodukt, das ohne Reinigung weiterverarbeitet wird. Nach DC/NMR entsteht ein Isomeres im Überschuß.

NMR ($CDCl_3$) : δ = 7,16(1)s, 7,12(1)s, 4,45(2)m, 3,10(3)s,
Hauptprodukt 1,52(9)s, 1,22(2)m, 0,03(9)s ppm.

## Beispiel 3

Z-2-(2-tert.-Butoxycarbonylaminothiazol-4-yl)-3-methylsulfonylpropensäure

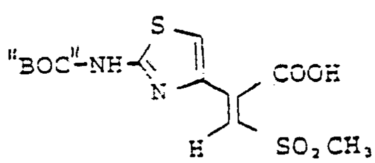

10,3 g des reinen, nach Beispiel 1 hergestellten Z-Isomeren werden in 50 ml Ethanol gelöst. Man fügt 60 ml 1 n Natronlauge zu und rührt über Nacht bei Raumtemperatur. Nach Abziehen des Alkohols wird die wäßrige Lösung bei pH 9,5 dreimal mit Essigester extrahiert, auf pH 2,5 gestellt und erneut dreimal mit Essigester extrahiert. Diese letzten vereinigten Extrakte trocknet man über Magnesiumsulfat und dampft ein. Kristallisation aus Toluol liefert reines Produkt. Fp. 135-40° (Zers.), Ausbeute 28,2 %.

Zu einer Lösung von 40,3 g des Produkts aus Beispiel 2 in 200 ml abs. THF gibt man 200 ml einer 1 m Lösung von Tetrabutylammoniumfluorid in THF. Nach einer Stunde Stehen bei Raumtemperatur wird das THF abgezogen, der Rückstand in Essigester gelöst und diese Lösung dreimal mit 1 n Schwefelsäure und einmal mit Wasser extrahiert. Trocknen, Eindampfen und Kristallisation mit Toluol liefert wird oben isomerenreines Produkt. Fp. 141,5 (Zers.), Ausbeute 28,1 % über 2 Stufen.

IR (Nujol) : 1 725, 1 595, 1 550, 1 460, 1 370, 1 305, 1 235, 1 200, 1 150, 1 120, 1 080 $cm^{-1}$.

NMR ($CDCl_3$) : δ = 9,25(2)s breit, 7,45(1)s, 7,27(1)s, 7,23(1)s, 3,23(3)s, 1,60(9)s ppm.

| | | | | |
|---|---|---|---|---|
| ber. für 1 Mol Produkt + 1/2 Mol Toluol : | C 47,2 | H 5,1 | N 7,1 | S 16,2 |
| gef. : | C 48,0 | H 5,2 | N 7,2 | S 15,8 |

Beispiel 4

Tert.-butyl-7-(2-(2-tert.-butoxycarbonylaminothiazol-4-yl)-3-methylsulfonylprop-2-enoyl)-amino-2-acetoxymethyl-3-cefem-4-carboxylat

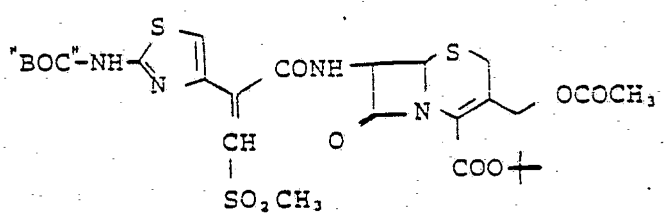

In einer ausgeheizten Apparatur werden unter Stickstoff 2 g der nach Beispiel 3 hergestellten Säure mit 0,71 ml Triethylamin in 200 ml Methylenchlorid gelöst. Man kühlt auf —60°, versetzt mit 0,39 ml Methansulfonsäurechlorid und rührt zwei Stunden nach. Dann werden 1,66 g 7-Aminocefalosporansäure-tert.-butylester, gelöst in 200 ml Methylenchlorid, zugegeben. Man läßt das Reaktionsgemisch langsam auf Raumtemperatur auftauen und rührt über Nacht nach.

Das Lösungsmittel wird kalt abgedampft, der Rückstand in Essigester aufgenommen und die organische Phase nacheinander dreimal mit 1 n Salzsäure, einmal mit Wasser, zweimal mit gesättigter Natriumbicarbonatlösung und noch einmal mit Wasser gewaschen, getrocknet und eingedampft. Es verbleiben 1,4 g eines gelben Schaums, der nach DC und NMR-Spektrum das gewünschte Z-Isomere in etwa 90 %iger Reinheit darstellt und daher ohne weitere Reinigung in die nächste Reaktion eingesetzt werden kann, Roh-Ausbeute 39,6 %. Zur Erzielung höherer Reinheit ist eine Chromatographie erforderlich.

Führt man die Aktivierung statt bei —60° bei —20° durch, so entsteht durch Equilibrierung der Doppelbindung ein ~ 1 : 1-E/Z-Gemisch, das chromatographisch getrennt werden kann (0,9 g an 80 g Kieselgel 60, 230-400 mesh, Merck, Laufmittel Methylenchlorid/Methanol 94/4).

Z-Isomeres

IR ($CHCl_3$) : 1 786, 1 727, 1 685, 1 551, 1 372, 1 318, 1 208, 1 155 $cm^{-1}$.

NMR ($CDCl_3$) : δ = 9,10(1)s, 7,80(1)d, J = 8 Hz, 7,27(1)s, 7,09(1)s, 6,02(1)dd, J = 8 Hz, 5,08(1)d, J = 5 Hz, 5,10(1)d, J = 23 Hz, 4,81(1)d, J = 13 Hz, 3,59(1), J = 18 Hz, 3,41(1)d, J = 18 Hz, 3,12(3)s, 2,10(3)s, 1,55(9)s, 1,53(9)s ppm.

E-Isomeres

IR ($CHCl_3$) : 1 775, 1 722, 1 676, 1 551, 1 395, 1 371, 1 313, 1 251, 1 206, 1 152 $cm^{-1}$.

NMR ($CDCl_3$) : δ = 10,82(1)s breit, 8,15(1)d, J = 8 Hz, breit, 7,81(1)s, 7,52(1)s, 5,43(1)dd, J = 8 Hz, J = 5 Hz, 4,88(1)d, J = 5 Hz, 5,21(1)d, J = 13 Hz, 4,67(1)d, J = 13 Hz, 3,50(1)d, J = 28 Hz, 3,35(1)d, J = 18 Hz, 2,94(3)s, 2,07(3)s, 1,55(9)s, 1,45(9)s ppm.

Beispiel 5

7-(2-(2-Aminothiazol-4-yl)-3-methylsulfonylprop-2-enoyl)-amino-3-acetoxymethyl-3-cefem-4-carbonsäuretrifluoracetat

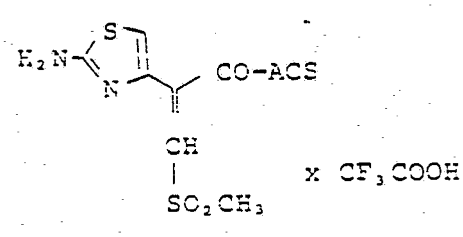

1. Z-Isomeres

0,8 g des in Beispiel 4 hergestellten Z-Isomeren werden unter Stickstoff bei 0° in 10 ml Trifluoressigsäure gelöst. Man läßt auf Raumtemperatur kommen und dampft nach einer Stunde kalt ein. Der Rückstand wird zweimal mit je 50 ml Methylenchlorid verrührt und die Lösung erneut eingedampft. Kristallisation mit Ether liefert das reine Produkt. Ausbeute 0,8 g.

IR (KBr) : 1 781, 1 728, 1 653, 1 542, 1 381, 1 316, 1 261, 1 198, 1 133, 1 102, 1 024 $cm^{-1}$.

NMR ($CD_3OD$) : δ = 7,02(1)s, 5,89(1)d, J = 5 Hz, 5,18(1)d, J = 5 Hz, 5,11(1)d, J = 13 Hz, 4,81(1)d, J = 12 Hz, 3,70(1)d, J = 18 Hz, 3,47(1)d, H = 18 Hz, 3,10(3)s, 2,03(3)s ppm.

2. E-Isomeres

Das nach Beispiel 4 hergestellte reine E-Isomere wird, wie unter 1. beschrieben, in die Titelverbindung überführt.

IR (KBr) : 1 782, 1 729, 1 670, 1 532, 1 383, 1 313, 1 263, 1 199, 1 140, 1 027 $cm^{-1}$.

NMR ($CD_3OD$) : δ = 7,39(1)s, 7,14(1)s, 5,81(1)d, J = 5 Hz, 5,13(1)d, J = 5 Hz, 5,08(1)d, J = 13 Hz, 4,81(1)d, J = 13 Hz, 3,68(1)d, J = 18 Hz, 3,46(1)d, J = 18 Hz, 3,15(3)s, 2,03(3)s ppm.

## Beispiel 6

Z-tert.-butyl-7-(2-(2-tert.-butoxycarbonylaminothiazol-4-yl)-3-methylsulfonylprop-2-enoyl)-amino-3-(1-methyl-1-H-tetrazol-5-yl)-thiomethyl-3-cefem-4-carboxylat

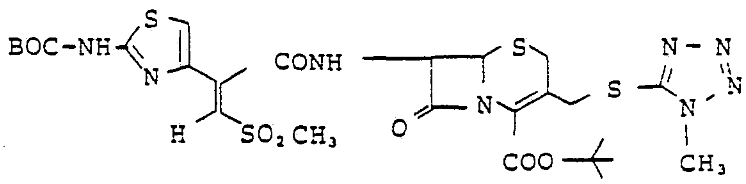

1 g der in Beispiel 3 hergestellten Säure werden analog zu Beispiel 4 mit 7-Amino-3-(1-methyl-1-H-tetrazol-5-yl)-thiomethylcefalosporansäure-tert.-butylester anstelle von 7-Aminocefalosporansäure-tert.-butylester umgesetzt. Man erhält 1 g Rohprodukt, das ohne Reinigung weiter verwendet werden kann.

IR (KBr) : 1 782, 1 715, 1 540, 1 452, 1 366, 1 243, 1 152, 1 133 $cm^{-1}$.

NMR ($CDCl_3$) : δ = 8,47(1)s, breit, 7,25(1)s, 7,23(1)d, J = 8 Hz, 7,05(1)s, 6,02(1)dd, J = 8 Hz, J = 5 Hz, 5,09(1)d, J = 5 Hz, 4,45(1)d, J = 13 Hz, 4,34(1)d, J = 13 Hz, 3,94(3)s, 3,72(2)s, 3,11(3)s, 1,56(18)s ppm.

## Beispiel 7

Z-7-(2-(2-Aminothiazol-4-yl)-3-methylsulfonylprop-2-enoyl)-amino-3-(1-methyl-1-H-tetrazol-5-yl)-thiomethyl-3-cefem-4-carbonsäure-trifluoracetat

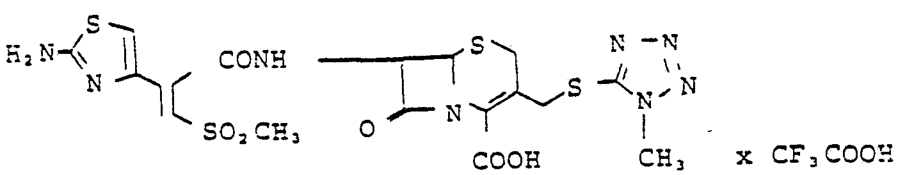

Behandlung des in Beispiel 6 hergestellten Produkts mit Trifluoressigsäure wie in Beispiel 5 beschrieben, führt zur Titelverbindung.

NMR ($CD_3OD$) : δ = 7,03(1)s, 7,02(1)s, 5,87(1)d, J = 5 Hz, 5,81(1)d, J = 5 Hz, 4,39(1)d, J = 15 Hz, 4,26(1)d, J = 15 Hz, 4,00(3)s, 3,85(1)d, J = 18 Hz, 3,70(1)d, J = 18 Hz, 3,12(3)s ppm.

## Beispiel 8

2-(2-Tert.-butoxycarbonylaminothiazol-4-yl)-3-methyl-sulfonylpropensäurediphenylmethylester

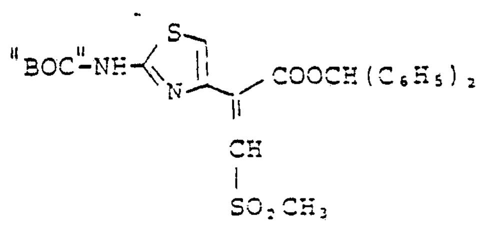

Analog zu Beispiel 2 werden 8,8 g Diethylmethylsulfonylmethylphosphonat mit 18 g 2-(2-Tert.-butoxycarbonylaminothiazol-4-yl)-2-oxoessigsäurediphenylmethylester umgesetzt. Aus dem Rohprodukt werden durch Kristallisation mit Ether 9,2 g des polaren Isomeren abgetrennt. Nach Eindampfen der Mutterlauge verbleiben 11,2 g des leicht verunreinigten unpolaren Isomeren als zähes Öl. Ausbeute 95 %.

polares Isomeres Fp. 191° (Z)

IR (Nujol) : 1 720, 1 600, 1 560, 1 460, 1 370, 1 315, 1 285, 1 240, 1 190, 1 160, 1 134, 1 080, 960 $cm^{-1}$.
NMR ($CDCl_3$) : δ = 8,20(1) breit, 7,43(10)s, 7,32(1)s, 7,18(1)s, 6,69(1)s, 2,94(3)s, 1,56(9)s ppm.

unpolares Isomeres

NMR ($CDCl_3$) : δ = 7,31(13)m, 3,03(3)s, 1,49(9)s ppm.

## Beispiel 9

2-(2-Aminothiazol-4-yl)-3-methylsulfonylpropensäure

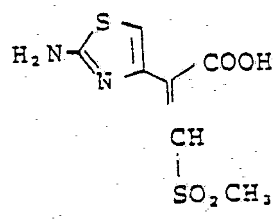

16 g des in Beispiel 8 hergestellten kristallinen Isomeren werden unter Stickstoffatmosphäre bei 0° in eine gerührte Lösung von 10,8 g Anisol in 200 ml Trifluoressigsäure eingetragen. Man läßt die klare Lösung auf Raumtemperatur kommen, rührt eine Stunde nach und dampft kalt ein. Der Rückstand wird zweimal in je 200 ml Methylenchlorid aufgenommen und die Lösung erneut eingedampft. Man gibt Essigester und Wasser zu und stellt mit 2 n Natronlauge pH 8 ein. Nach Abtrennen der organischen Phase wird die wäßrige Phase noch zweimal mit Essigester gewaschen, dann mit Essigester versetzt und durch Zugabe von 2 n Salzsäure auf pH 2 zurückgestellt. Das ausgefallene Produkt wird abgesaugt und aus Acetonitril umkristallisiert. Ausbeute 54,4 %, Fp. 181° (Zers.).
IR (Nujol) : 1 665, 1 600, 1 580, 1 315, 1 295, 1 260, 1 130, 970 $cm^{-1}$.
NMR (DMSO) : δ = 7,34(2)s, 6,95(1)s, 6,87(1)s, 3,12(3)s ppm.

| | | | | |
|---|---|---|---|---|
| ber. : | C 33,9 | H 3,2 | N 11,3 | S 25,8 |
| gef. : | C 33,9 | H 3,4 | N 11,6 | S 23,9 |

## Beispiel 10

Natrium-6-(2-(2-aminothiazol-4-yl)-3-methylsulfonylpropenoyl)-aminopenam-3-carboxylat

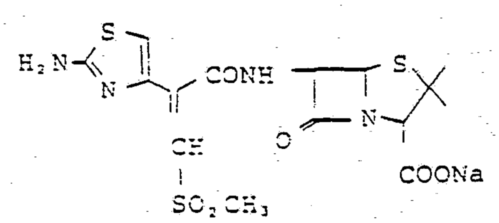

Zu einer Lösung von 1 g des nach Beispiel 9 hergestellten Produkts und 1,2 ml Tributylamin in 50 ml absolutem Methylenchlorid werden unter Stickstoffatmosphäre bei — 50° 0,4 ml Methansulfonsäurechlorid gegeben. Man rührt 24 h bei — 78° nach und tropft dann eine Lösung von 1,2 g 6-Aminopenicillansäure und 1,5 ml Triethylamin in 50 ml Methylenchlorid zu. Nach einer Stunde taut man die Lösung auf, rührt 20 Stunden bei Raumtemperatur nach und dampft dann kalt ein. Der Rückstand wird in Wasser aufgenommen, die Lösung auf pH 2,8 eingestellt und dreimal mit Essigester extrahiert. Die vereinigten organischen Phasen wäscht man mit Wasser, gibt dann Wasser zu und stellt mit 2 n Natronlauge langsam pH 7,0 ein. Nach Abtrennen der organischen Phase werden durch Gefriertrocknung der wäßrigen Lösung 0,8 g des gewünschten Produkts als Isomerengemisch erhalten.
IR (Nujol) : 1 780, 1 650, 1 600, 1 540, 1 305, 1 135, 975 $cm^{-1}$.
NMR (DMSO) : δ = 9,26/9,58(1)d, J = 6 Hz, 7,33(2)s verbreitert, 6,98(1)s, 6,87/6,82(1)s, 5,54(2)m,

4,25/4,24(1)s, 3,14/3,12(3)s, 1,58(3)s, 1,48(3)s ppm.

Beispiel 11

2-(2-tert.-Butoxycarbonylaminothiazol-4-yl)-3-phenyl-sulfonylpropensäure-(2-trimethylsilylethyl) ester

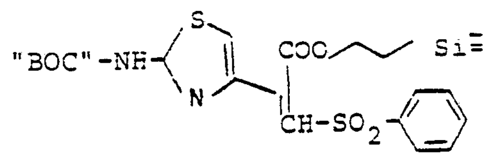

16,0 g Diethylphenylsulfonylmethylphosphonat werden analog zu Beispiel 2 mit 20,4 g 2-(2-tert-Butoxycarbonylaminothiazol-4-yl)-2-oxoessigsäure-(2-trimethylsilylethyl) ester umgesetzt. Man erhält 32,6 g eines öligen Rohprodukts, aus dem durch Chromatographie an Kieselgel (Laufmittel Toluol/Essigester : 7/1) 7,8 g des unpolaren Z-Isomeren und 5,2 g des polaren E-Isomeren abgetrennt werden.

Z-Isomeres

NMR (DMSO) : δ = 11,74 [1] s, 7,95 [2] m, 7,70 [3] m, 7,57 [1] s, 7,03 [1] s, 4,43 [2] m, 1,43 [9] s, 1,11 [2] m, 0,02 [9] s ppm.

E-Isomeres

NMR (DMSO) : δ = 11,51 [1] s, 7,85 [2] m, 7,55-7,80 [3] m, 7,45 [1] s, 7,36 [1] s, 4,21 [2] m, 1,49 [9] s, 0,92 [2] m, —0,05 [9] s ppm.

Beispiel 12

2-(2-tert.-Butoxycarbonylaminothiazol-4-yl)-3-phenylsulfonylpropensäure

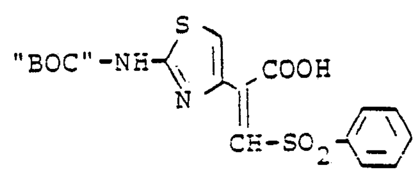

7,2 g des nach Beispiel 11 hergestellten Z-Isomeren werden bei Raumtemperatur in 28,8 ml einer 1 N-Lösung von Tetrabutylammoniumfluorid in THF gelöst. Man rührt die Lösung über Nacht und arbeitet dann auf wie in Beispiel 3 beschrieben.

Kristallisation mit Ether liefert 5,1 g Säure in Form des reinen Z-Isomeren. Fp. 194° (Z).

IR (Nujol) : 1 700, 1 600, 1 555, 1 300, 1 280, 1 239, 1 140, 1 072 $cm^{-1}$.

NMR (DMSO) : δ = 11,74 [1] s, 7,98 [2] m, 7,70 [3] m, 7,55 [1] s, 6,96 [1] s, 1,47 [9] s ppm.

Auf die gleiche Weise wird aus dem nach Beispiel 11 hergestellten E-Isomeren die E-Carbonsäure hergestellt.

IR ($CHCl_3$) : 1 710, 1 540, 1 440, 1 360, 1 295, 1 223, 1 141, 1 074 $cm^{-1}$.

NMR (DMSO) : δ = 11,52 [1] s, 7,87 [2] m, 7,55-7,80 [3] m, 7,45 [1] s, 7,32 [1] s, 1,50 [9] s ppm.

Beispiel 13

tert.-butyl-7-(2-(2-tert.-butoxycarbonylaminothiazol-4-yl)-3-phenylsulfonylprop-2-enoyl) amino-3-acetoxymethyl-3-cefem-4-carboxylat

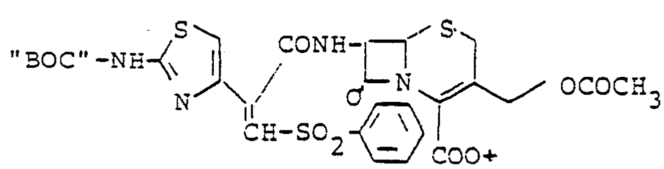

1,25 g des nach Beispiel 12 hergestellten Z-Isomeren werden gemäß der Vorschrift von Beispiel 4 mit

7-Aminocefalosporansäure-tert.-butylester umgesetzt. Man erhält 1,6 g eines gelben Schaums, der ohne weitere Reinigung umgesetzt wird.

IR ($CHCl_3$) : 1 779, 1 715, 1 540, 1 360, 1 300, 1 218, 1 141 $cm^{-1}$

NMR (DMSO) : δ = 9,88 [1] d, J = 7 Hz, 8.02 [2] d, J = 7 Hz, 7,60-7,84 [3] m, 7,34 [1] s, 6,88 [1] S, 5,96 [1] dd, J = 7 Hz, J = 5 Hz, 5,30 [1] d, J = 5 Hz, 4,98 [1] d, J = 13 Hz, 4,66 [1] d, J = 13 Hz, 3,70 [1] d, J = 17 Hz, 3,54 [1] d, J = 17 Hz, 2,06 [3] s, 1,51 [9] s, 1,49 [9] s ppm.

Analog werden 1,25 g des nach Beispiel 12 hergestellten E-Isomeren umgesetzt :

IR (KBr) : 1 778, 1 721, 1 675, 1 544, 1 447, 1 392, 1 369, 1 250, 1 153 $cm^{-1}$.

NMR (DMSO) : δ = 11,60 [1] s, 9,54 [1] d, J = 7 Hz, 7,92 [2] d, J = 7 Hz, 7,55-7,80 [3] m, 7,48 [1] s, 7,10 [1] s, 5,71 [1] dd, J = 7 Hz, J = 5 Hz, 5,18 [1] d, J = 5 Hz, 4,92 [1] d, J = 13 Hz, 4,64 [1] d, J = 13 Hz, 3,69 [1] d, J = 17 Hz, 3,42 [1] d, J = 17 Hz, 2,05 [3] s, 1,49 [9] s ppm.

Beispiel 14

Natrium-7-(2-(2-Aminothiazol-4-yl)-3-phenylsulfonylprop-2-enoyl)-amino-3-acetoxymethyl-3-cefem-4-carboxylat

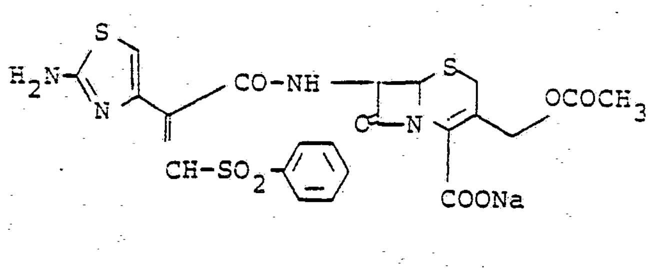

1.Z-Isomeres

1,5 g des nach Beispiel 13 hergestellten Z-Isomeren werden bei 0 °C in 50 ml Trifluoressigsäure gelöst. Nach einer Stunde Rühren bei Raumtemperatur dampft man kalt ein, versetzt den Rückstand mit je 50 ml absolutem Methylenchlorid und dampft erneut ein. Der Rückstand wird nun in 10 ml Wasser unter Zugabe von 2 N Natronlauge bei pH 7 gelöst und die Lösung dreimal mit Essigester extrahiert. Man fällt durch Zugabe von 2 N Salzsäure bei pH 3,0 die freie Säure aus, saugt ab, suspendiert den Rückstand in Wasser und bringt durch Zugabe von 2 N-Natronlauge bei pH 7,0 wieder in Lösung. Lyophilisieren der wäßrigen Phase liefert 1 g Produkt.

IR (Nujol) : 1 780, 1 675, 1 605, 1 530, 1 300, 1 230, 1 150, 1 090, 1 030 $cm^{-1}$.

NMR ($CD_3OD$) : δ = 8,03 [2] d, J = 6 Hz, 7,62 [3] m, 6,98 [1] s, 6,95 [1] s, 5,87 [1] d, J = 5 Hz, 5,17 [1] d, J = 5 Hz, 5,01 [1] d, J = 13 Hz, 4,81 [1] d, J = 13 Hz, 3,64 [1] d, J = 18 Hz, 3,35 [1] d, J = 18 Hz, 2,03 [3] s ppm.

2.E-Isomeres

Auf gleiche Weise erhält man aus dem E-Isomeren des Beispiels 13 das E-Isomere der Titelverbindung.

IR (Nujol) : 1 760, 1 720, 1 603, 1 300, 1 240, 1 150, 1 075, 1 028 $cm^{-1}$.

NMR ($CD_3OD$) : δ = 7,83 [2] m, 7,50-7,72 [3] m, 7,32 [1] s, 7,08 [1] s, 5,71 [1] d, J = 5 Hz, 5,05 [1] d, J = 5 Hz, 4,99 [1] d, J = 13 Hz, 4,82 [1] d, J = 13 Hz, 3,59 [1] d, J = 17 Hz, 3,32 [1] d, J = 17 Hz, 2,04 [3] s ppm.

Beispiel 15

2-(2-tert.-Butoxycarbonylaminothiazol-4-yl)-3-methylsulfinyl-propensäure-(2-trimethylsilylethyl) ester

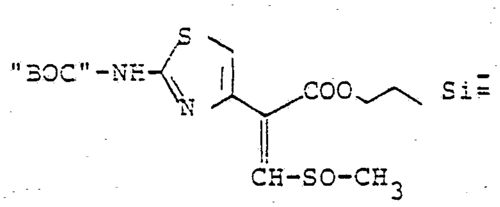

5,25 g Diethylmethylsulfinylmethylphosphonat werden analog zu Beispiel 2 mit 7,4 g 2-(2-tert.-Butoxycarbonylaminothiazol-4-yl)-2-oxoessigsäure-(2-trimethylsilylethyl)-ester umgesetzt. Chromatografie des Rohprodukts an Kieselgel (Laufmittel Toluol/Essigsäure : 4/1) liefert 3 g des Z-Isomeren und 2,8 g des E-Isomeren.

Z-Isomeres

NMR (DMSO) : δ = 11,71 [1] s, 7,47 [1] s, 7,31 [1] s, 4,35 [2] m, 2,78 [3] s, 1,48 [9] s, 1,09 [2] m, 0,03 [9] s ppm.

E-Isomeres

NMR (DMSO) : δ = 11,66 [1] s, 7,62 [1] s, 7,13 [1] s, 4,32 [2] m, 2,87 [3] s, 1,50 [9] s, 1,07 [2] m, 0,03 [9] ppm.

Beispiel.16

2-(2-tert.-Butoxycarbonylaminothiazol-4-yl)-3-methylsulfinyl-propensäure

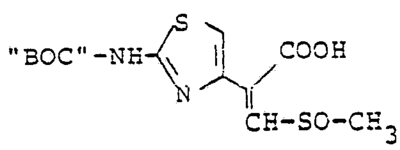

Die nach Beispiel 15 hergestellten isomerenreinen Ester werden gemäß Beispiel 12 getrennt umgesetzt und aufgearbeitet.

E-Isomeres

Fp. : 182° (Z) (Ether)
IR (KBr) : 1 722, 1 546, 1 453, 1 393, 1 366, 1 279, 1 245, 1 155, 1 127, 1 077 $cm^{-1}$.
NMR (DMSO) : δ = 10,02 [1] s, 7,64 [1] s, 7,18 [1] s, 2,90 [3] s, 1,50 [9].

Z-Isomeres

Fp. : 169° (Z) (Methylenchlorid)
IR (KBr) : 1 720, 1 560, 1 454, 1 370, 1 246, 1 154, 1 075 $cm^{-1}$.
NMR (DMSO) : δ = 9,94 [1] s, 7,50 [1] s, 7,28 [1] s, 2,73 [3] s, 1,49 [9] s ppm.

Beispiel 17

E-tert.-butyl-7-(2-(2-tert.-butoxycarbonylaminothiazol-4-yl)-3-methylsulfinylprop-2-enoyl)-amino-3-acetoxymethyl-3-cefem-4-carboxylat

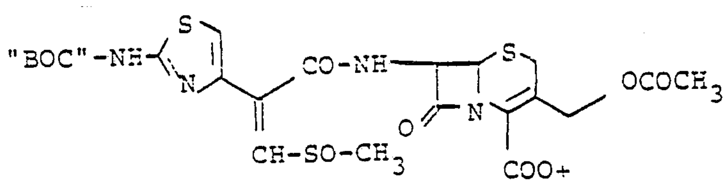

1 g des nach Beispiel 16 hergestellten E-Isomeren wird gemäß der Vorschrift aus Beispiel 4 mit 7-Aminocefalosporansäure-tert.-butylester umgesetzt. Das Rohprodukt wird an Kieselgel chromatografiert (Laufmittel Essigester). Man erhält 320 mg eines Doppelbindungsisomeren, dessen NMR-Signale infolge der Sulfoxid-Isomerie zum Teil aufgespalten sind.

IR (KBr) : 1 787, 1 722, 1 671, 1 551, 1 452, 1 370, 1 247, 1 154, 1 072, 1 016 $cm^{-1}$.

NMR ($CDCl_3$) : δ = 9,0 [2] m, 7,31/7,32 [1] s, 7,24/7,17 [1] s, 5,78 [1] dd, J = 5 Hz, J = 8 Hz, 5,14/5,12 [1] d, J = 13 Hz, 5,07/5,03 [1] d, J = 5 Hz, 4,97/4,73 [1] d, J = 13 Hz, 3,55 [1] d, J = 17 Hz, 3,37 [1] d, J = 17 Hz, 2,88/2,82 [3] s, 2,09/2,08 [3] s, 1,57/1,56 [9] s, 1,55 [9] s ppm.

Beispiel 18

E-7-(2-(2-Aminothiazol-4-yl)-3-methylsulfinylprop-2-enoyl)-amino-3-acetoxymethyl-3-cefem-4-carbonsäure-tri-fluoracetat

(Siehe Formel Seite 16 f.)

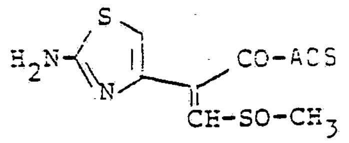 x $CF_3COOH$

90 mg des Produkts aus Beispiel 17 werden unter Stickstoff bei 0 °C in 2 ml Trifluoressigsäure, die 14,4 µl Anisol enthalten, gelöst. Man läßt auf Raumtemperatur kommen und dampft nach einer Stunde kalt ein. Der Rückstand wird zweimal mit je 10 ml abs. Methylenchlorid verrührt und die Lösung erneut eingedampft. Kristallisation mit Ether liefert 40 mg des reinen Produkts.

IR (KBr) : 1 772, 1 723, 1 665, 1 525, 1 379, 1 232, 1 064, 1 025 $cm^{-1}$.

NMR ($CD_3OD$) : δ = 6,97 [1] s, 6,71 [1] s, 5,81 [1] dd, J = 4 Hz, J = 5 Hz, 5,16 [1] d, J = 4 Hz, 5,12 [1] d, J = 12 Hz, 4,76 [1] d, J = 12 Hz, 3,70 [1] d, J = 18 Hz, 3,50 [1] d, J = 18 Hz, 2,95 [3] s, 2,04 [3] s ppm.

Beispiel 19

Z-7-(2-(2-Aminothiazol-4-yl)-3-methylsulfonylprop-2-enoyl)-amino-3-pyridiniummethyl-3-cefem-4-carboxylat

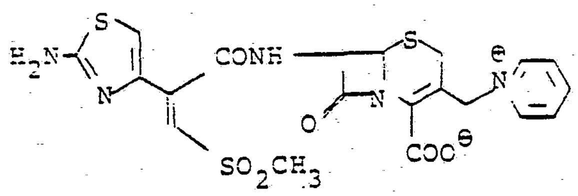

Eine Lösung von 0,65 g des Produkts aus Beispiel 9 in 4,5 ml absolutem Dimethylformamid wird unter Stickstoffatmosphäre mit 0,35 g 1-Hydroxybenztriazol und 0,55 g Dicyclohexylcarbodiimid versetzt. Man rührt 4,5 Stunden bei Raumtemperatur und setzt dann eine Lösung von 0,75 g 7-Amino-3-pyridiniummethyl-3-cefem-4-carboxylat in 2,5 ml Wasser zu. Nach Rühren über Nacht wird vom ausgefallenen Harnstoff abgesaugt und das Filtrat kalt eingeengt. Man nimmt den verbleibenden Rückstand bei pH 7 in Wasser auf, extrahiert die Lösung dreimal mit Essigester, stellt die wäßrige Phase mit 2 N-Salzsäure auf pH 3 und extrahiert erneut mit Essigester. Nachdem die wäßrige Phase mit 2 N-Natronlauge auf pH 7 gestellt und lyophilisert wurde, wird aus dem Lyophilisat die Titelverbindung durch Chromografie rein abgetrennt. Ausbeute 80 mg eines Produkts, das geringe Mengen des E-Isomeren enthält.

IR (KBr) : 1 767, 1 661, 1 616, 1 535, 1 486, 1 398, 1 299, 1 227, 1 133, 1 024, 970 $cm^{-1}$.

NMR ($D_2O$) : δ = 8,82 [2] d, J = 6 Hz, 8,44 [1] t, J = 6 Hz, 7,96 [2] t, J = 6 Hz, 7,00 [1] s, 6,93 [1] s, 5,82 [1] d, J = 5 Hz, 5,43 [1] d, J = 14 Hz, 5,25 [1] d, J = 14 Hz, 5,15 [1] d, J = 5 Hz, 3,53 [1] d, J = 18 Hz, 3,11 [3] s, 3,09 [1] d, J = 18 Hz ppm.

**Patentansprüche**

1. Verbindungen der allgemeinen Formel (I)

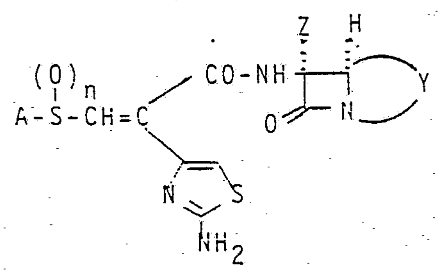

(I)

in der

Z Wasserstoff oder $C_1$-$C_6$-Alkoxy,

n 1 oder 2,

Y in der Form der freien Säure einen Rest der Formel IIa oder IIb

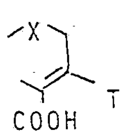

(IIa)

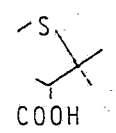

(IIb)

bedeutet, worin

X —S—, —SO— oder —$SO_2$— und

T ein in der Cephalosporinchemie üblicher Rest ist,

und worin

A Hydroxy, Alkyl mit bis zu 18 C-Atomen, das gegebenenfalls durch Halogen, Hydroxy, $C_1$-$C_4$-Alkoxy, $OCOR^2$, Thio, eine Gruppe

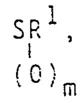

eine Gruppe

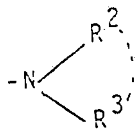

Nitro, Cyano, Hydroxycarbonyl, $C_1$-$C_4$-Alkoxycarbonyl, Aminocarbonyloxy, Sulfo oder einen 5- oder 6-gliedrigen heterocyclischen Ring substituiert ist, wobei

$R^1$ verzweigtes oder unverzweigtes $C_1$-$C_4$-Alkyl bedeutet,

m 0, 1 oder 2 sein kann und

$R^2$ und $R^3$ gleich oder verschieden sind und Wasserstoff, $C_1$-$C_5$-Alkyl oder $C_1$-$C_5$-Alkanoyl sein können, einen Rest der Formel

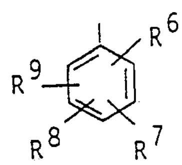

worin $R^6$, $R^7$, $R^8$ und $R^9$ unabhängig voneinander Wasserstoff, Halogen, Alkyl mit bis zu 6 Kohlenstoffatomen, Aryl, eine Gruppe —$OCOR^{10}$, eine Gruppe

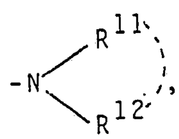

Nitro, Cyano, ($C_1$-$C_4$)-Alkoxy, ($C_1$-$C_4$)-Alkyl-thio, Hydroxycarbonyl, ($C_1$-$C_4$)-Alkoxy-carbonyl, Aminocarbonyloxy, Sulfonyl oder Sulfo bedeuten, worin wiederum Aryl einen 5- oder 6-gliedrigen heterocyclischen Ring bedeutet, und worin $R^{10}$ verzweigtes oder unverzweigtes Alkyl mit bis zu 6 Kohlenstoffatomen bedeutet, und worin $R^{11}$ und $R^{12}$ gleich oder verschieden sind und Wasserstoff, Alkyl oder Alkanoyl sein können, jeweils mit bis zu 6 C-Atomen im Alkylteil, oder einen heterocyclischen 5- oder 6-Ring mit Stickstoff, Sauerstoff oder Schwefel als Heteroatomen im Ring bedeutet, sowie ihre nicht-toxischen pharmazeutisch verträglichen Salze.

2. Verbindungen nach Anspruch 1, in denen n = 2 ist.

3. Verbindungen nach Anspruch 2 in der Z-Konfiguration.

4. Verbindungen nach den Ansprüchen 1 bis 3, worin

A einen gegebenenfalls wie in Anspruch 1 angegeben substituierten Alkylrest mit bis zu 12 C-Atomen oder einen gegebenenfalls wie in Anspruch 1 angegeben substituierten Phenylrest,

X Schwefel,

T Wasserstoff, $C_1$-$C_4$-Alkyl, Halogen, $C_1$-$C_4$-Alkoxy, Hydroxymethyl, Formyloxymethyl, $C_1$-$C_4$-Alkylcarbonyloxymethyl, Aminocarbonyloxymethyl, Pyridiniummethyl, 4-Carbamoylpyridiniummethyl, Halogenmethyl, Dichloracetoxymethyl, Sulfonyloxymethyl oder Heterocyclylthiomethyl bedeutet.

5. Verbindungen nach den Ansprüchen 1 bis 4, in denen

Y die Bedeutung IIb oder IIa hat,

worin in der Bedeutung von IIa

X Schwefel,

T Wasserstoff, Halogen oder einen Rest

$-CH_2OCOCH_3$ , 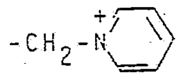 , 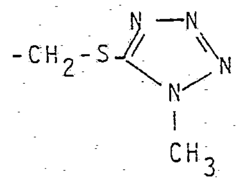 ,

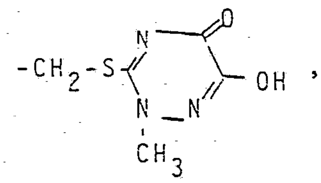 , 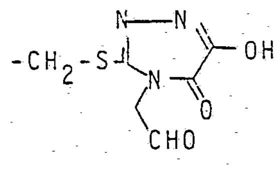 oder

$-CH_2OCONH_2$ darstellt, und in denen

n 2 ist,

Z Wasserstoff oder Methoxy und

A Hydroxy, Alkyl mit bis zu 6 Kohlenstoffatomen oder einen unsubstituierten oder wie in Anspruch 1 angegeben substituierten Phenylrest bedeutet.

6. Verbindungen, die der Formel (III)

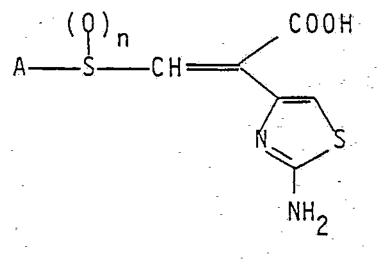

(III)

entsprechen, worin A und n die in Anspruch 1 angegebene Bedeutung besitzen.

7. Verbindungen gemäß Anspruch 6 in der Z-Konfiguration.

8. Verfahren zur Herstellung von Verbindungen der Formel (I)

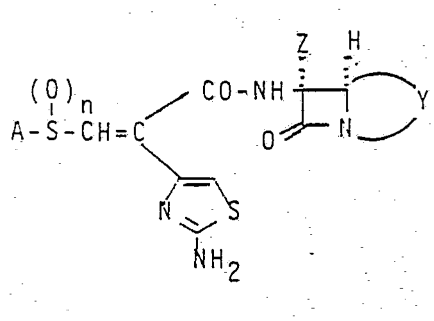

(I)

in der

Z Wasserstoff oder $C_1$-$C_6$-Alkoxy,

n 1 oder 2,

Y in der Form der freien Säure einen Rest der Formel IIa oder IIb

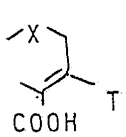

(IIa)

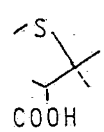

(IIb)

bedeutet, worin

X —S—, —SO— oder $—SO_2—$ und

T ein in der Cephalosporinchemie üblicher Rest ist,

und worin

A Hydroxy, Alkyl mit bis zu 18 C-Atomen, das gegebenenfalls durch Halogen, Hydroxy, $C_1$-$C_4$-Alkoxy, $OCOR^2$, Thio, eine Gruppe

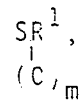

eine Gruppe

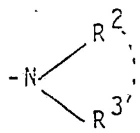

Nitro, Cyano, Hydroxycarbonyl, $C_1$-$C_4$-Alkoxycarbonyl, Aminocarbonyloxy, Sulfo oder einen 5- oder 6-gliedrigen heterocyclischen Ring substituiert ist, wobei

$R^1$ verzweigtes oder unverzweigtes $C_1$-$C_4$-Alkyl bedeutet,

m 0, 1 oder 2 sein kann und

$R^2$ und $R^3$ gleich oder verschieden sind und Wasserstoff, $C_1$-$C_5$-Alkyl oder $C_1$-$C_5$-Alkanoyl sein können, einen Rest der Formel

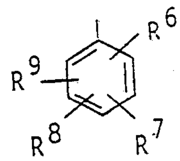

worin $R^6$, $R^7$, $R^8$ und $R^9$ unabhängig voneinander Wasserstoff, Halogen, Alkyl mit bis zu 6 Kohlenstoffatomen, Aryl, eine Gruppe —$OCOR^{10}$, eine Gruppe

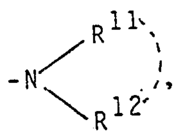

Nitro, Cyano, ($C_1$-$C_4$)-Alkoxy, ($C_1$-$C_4$)-Alkyl-thio, Hydroxycarbonyl ($C_1$-$C_4$)-Alkoxy-carbonyl, Aminocarbonyloxy, Sulfonyl oder Sulfo bedeuten, worin wiederum Aryl einen 5- oder 6-gliedrigen heterocyclischen Ring bedeutet, und worin $R^{10}$ verzweigtes oder unverzweigtes Alkyl mit bis zu 6 Kohlenstoffatomen bedeutet, und worin $R^{11}$ und $R^{12}$ gleich oder verschieden sind und Wasserstoff, Alkyl oder Alkanoyl sein können, jeweils mit bis zu 6 C-Atomen im Alkylteil, oder einen heterocyclischen 5- oder 6-Ring mit Stickstoff, Sauerstoff oder Schwefel als Heteroatomen im Ring bedeutet, dadurch gekennzeichnet, daß man

a) Verbindungen der Formel (III)

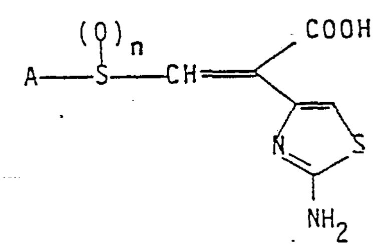

(III)

worin A und n die obengenannte Bedeutung haben, in an sich bekannter Weise mit Verbindungen der Formel (IV)

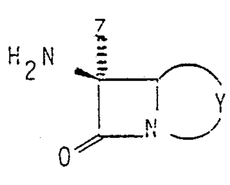

(IV)

in der Z und Y die obengenannte Bedeutung haben, umsetzt, oder

b) zum Erhalt von Verbindungen mit Z = $OCH_3$ solche Verbindungen der Formel (I), in den Z = H ist, in an sich bekannter Weise alkoxyliert oder

c) Verbindungen der Formel (I) mit Y in der Bedeutung IIa, in der T die Bedeutung $CH_2$—O—CO—Niedrigalkyl oder Halogenmethyl, Sulfonyloxymethyl oder Dichloracetoxymethyl hat, mit Nukleophilen umsetzt, oder

d) Verbindungen der Formel (I) mit Y in der Bedeutung von IIa, und T in der Bedeutung —$CH_2$—OH mit O=C=N—$SO_2$Cl oder O=C=N—$COCl_3$ umsetzt und anschließend die $SO_2$Cl oder $COCCl_3$-Gruppe abspaltet.

9. Verwendung der Verbindungen der allgemeinen Formel (I) nach Anspruch 8 zur Herstellung von Arzneimitteln.

**Claims**

1. Compounds of the general formula (I)

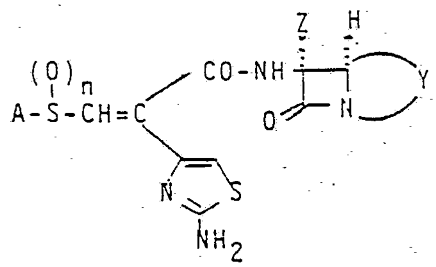

(I)

in which

Z denotes hydrogen or $C_1$-$C_6$-alkoxy,

n denotes 1 or 2,

Y, in the form of the free acid, denotes a radical of the formula IIa or IIb

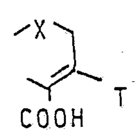

(IIa)

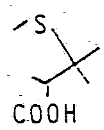

(IIb)

wherein

X is —S—, —SO— or —$SO_2$ and

T is a radical customary in cephalosporin chemistry, and wherein

A denotes hydroxyl, alkyl with up to 18 C atoms, which is optionally substituted by halogen, hydroxyl, $C_1$-$C_4$-alkoxy, $OCOR^2$, thio, a group

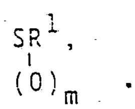

a group

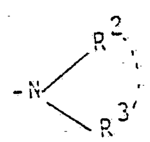

nitro, cyano, hydroxycarbonyl, $C_1$-$C_4$-alkoxycarbonyl, aminocarbonyloxy, sulpho or a 5- or 6-membered heterocyclic ring, wherein

$R^1$ denotes branched or unbranched $C_1$-$C_4$-alkyl,

m can be 0, 1 or 2 and

$R^2$ and $R^3$ are identical or different and can be hydrogen, $C_1$-$C_5$-alkyl or $C_1$-$C_5$-alkanoyl, a radical of the formula

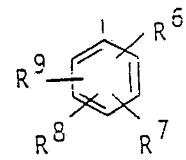

wherein $R^6$, $R^7$, $R^8$ and $R^9$ independently of one another denote hydrogen, halogen, alkyl with up to 6 carbon atoms, aryl, a group $—OCOR^{10}$, a group

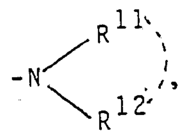

nitro, cyano, ($C_1$-$C_4$)-alkoxy ($C_1$-$C_4$)-alkyl-thio, hydroxycarbonyl, ($C_1$-$C_4$)-alkoxycarbonyl, aminocarbonyloxy, sulphonyl or sulpho, wherein aryl in turn denotes a 5- or 6-membered heterocyclic ring, and wherein $R^{10}$ denotes branched or unbranched alkyl with up to 6 carbon atoms, and wherein $R^{11}$ and $R^{12}$ are identical or different and can be hydrogen, alkyl or alkanoyl, each with up to 6 C atoms in the alkyl part, or a heterocyclic 5- or 6-ring with nitrogen, oxygen or sulphur as heteroatoms in the ring, and their non-toxic pharmaceutically tolerated salts.

2. Compounds according to Claim 1, in which n = 2.

3. Compounds according to Claim 2 in the Z configuration.

4. Compounds according to Claims 1 to 3, wherein

A denotes an alkyl radical which has up to 12 C atoms and is optionally substituted as mentioned in Claim 1 or a phenyl radical which is optionally substituted as mentioned in Claim 1,

X denotes sulphur,

T denotes hydrogen, $C_1$-$C_4$-alkyl, halogen, $C_1$-$C_4$-alkoxy, hydroxymethyl, formyloxymethyl, $C_1$-$C_4$-alkylcarbonyloxymethyl, aminocarbonyloxymethyl, pyridiniummethyl, 4-carbamoylpyridiniummethyl, halogenomethyl, dichloroacetoxymethyl, sulphonyloxymethyl or heterocyclylthiomethyl.

5. Compounds according to Claims 1 to 4, in which

Y has the meaning IIb or IIa,

wherein in the meaning IIa

X represents sulphur,

T represents hydrogen, halogen or a radical

$-CH_2OCOCH_3$ , 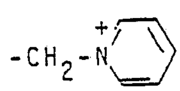 , 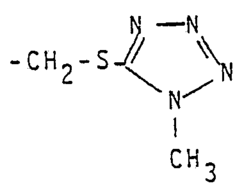 ,

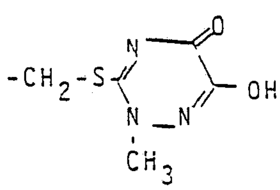 , 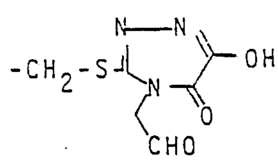 or

$—CH_2OCONH_2$, and in which

n is 2,

Z denotes hydrogen or methoxy and

A denotes hydroxyl, alkyl with up to 6 carbon atoms or a phenyl radical which is unsubstituted or substituted as mentioned in Claim 1.

6. Compounds which correspond to the formula (III)

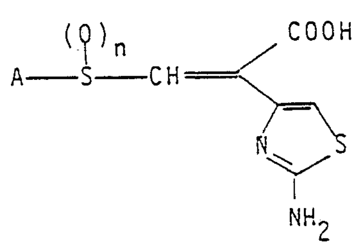

(III)

wherein A and n have the meaning given in Claim 1.

7. Compounds according to Claim 6 in the Z configuration.

8. Process for the preparation of compounds of the formula (I)

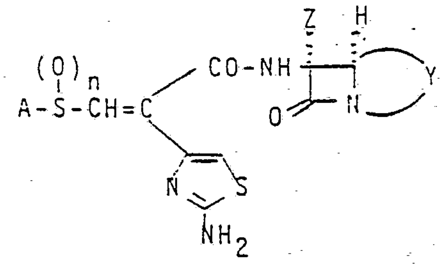

(I)

in which

Z denotes hydrogen or $C_1$-$C_6$-alkoxy,

n denotes 1 or 2,

Y, in the form of the free acid, denotes a radical of the formula IIa or IIb

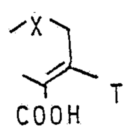

(IIa)

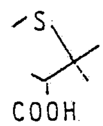

(IIb)

wherein

X is —S—, —SO— or —$SO_2$— and

T is a radical customary in cephalosporin chemistry, and wherein

A denotes hydroxyl, alkyl with up to 18 C atoms, which is optionally substituted by halogen, hydroxyl, $C_1$-$C_4$-alkoxy, $OCOR^2$, thio, a group

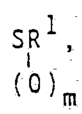

a group

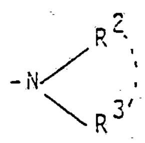

nitro, cyano, hydroxycarbonyl, $C_1$-$C_4$-alkoxycarbonyl, aminocarbonyloxy, sulpho or a 5- or 6-membered heterocyclic ring, wherein

$R^1$ denotes branched or unbranched $C_1$-$C_4$-alkyl,

m can be 0, 1 or 2 and

$R^2$ and $R^3$ are identical or different and can be hydrogen, $C_1$-$C_5$-alkyl or $C_1$-$C_5$-alkanoyl, a radical of the formula

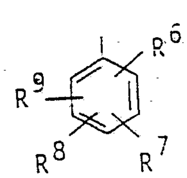

wherein $R^6$, $R^7$, $R^8$ and $R^9$ independently of one another denote hydrogen, halogen, alkyl with up to 6 carbon atoms, aryl, a group —$OCOR^{10}$, a group

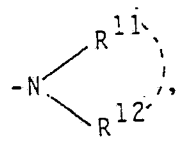

nitro, cyano, ($C_1$-$C_4$)-alkoxy, ($C_1$-$C_4$)-alkyl-thio, hydroxycarbonyl, ($C_1$-$C_4$-alkoxycarbonyl, aminocarbonyloxy, sulphonyl or sulpho, wherein aryl in turn denotes a 5- or 6-membered heterocyclic ring, and wherein $R^{10}$ denotes branched or unbranched alkyl with up to 6 carbon atoms, and wherein $R^{11}$ and $R^{12}$ are identical or different and can be hydrogen, alkyl or alkanoyl, each with up to 6 C atoms in the alkyl part, or a heterocyclic 5- or 6-ring with nitrogen, oxygen or sulphur as heteroatoms in the ring, characterised in that

a) compounds of the formula (III)

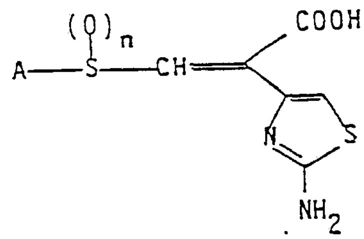

(III)

wherein A and n have the abovementioned meaning, are reacted, in a manner known per se, with compounds of the formula (IV)

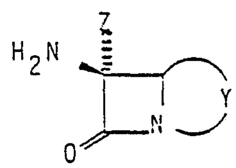

(IV)

in which Z and Y have the abovementioned meaning, or

b) to obtain compounds with $Z = OCH_3$, those compounds of the formula (I) in which Z is H are alkoxylated in a manner known per se or

c) compounds of the formula (I) in which Y has the meaning IIa, in which T has the meaning $CH_2$—O—CO— lower alkyl or halogenomethyl, sulphonyloxymethyl or dichloroacetoxymethyl, are reacted with nucleophiles, or

d) compounds of the formula (I) in which Y has the meaning of IIa and T has the meaning —$CH_2$—OH are reacted with O=C=N—$SO_2Cl$ or O=C=N—$COCl_3$ and then the $SO_2Cl$ or $COCCl_3$ group is split off.

9. Use of the compounds of the general formula (I) according to Claim 8 for the preparation of medicaments.

**Revendications**

1. Composés de formule générale (I) :

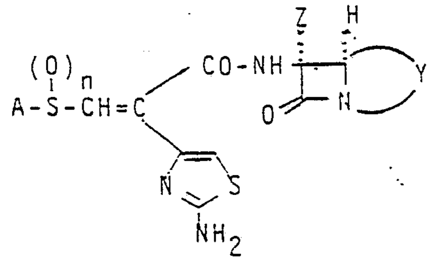

(I)

dans laquelle :

Z représente l'hydrogène ou un groupe alcoxy en $C_1$-$C_6$,

n est égal à 1 ou 2,

Y, à l'état d'acide libre, représente un reste de formule IIa ou IIb :

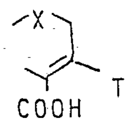 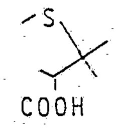

(IIa) (IIb)

dans lesquelles :

X représente —S—, —SO— ou —$SO_2$— et

T représente un reste usuel dans la chimie des céphalosporines, et

A représente un groupe hydroxy, alkyle contenant jusqu'à 18 atomes de carbone, éventuellement substitué par des halogènes, des groupes hydroxy, alcoxy en $C_1$-$C_4$, $OCOR^2$, thio, un groupe

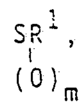

un groupe

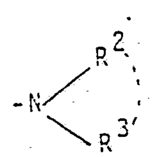

nitro, cyano, hydroxycarbonyle (alcoxy en $C_1$-$C_4$)-carbonyle, aminocarbonyloxy, sulfo ou un noyau hétérocyclique à 5 ou 6 chaînons,

$R^1$ représente un groupe alkyle en $C_1$-$C_4$ ramifié ou non,

m est égal à 0, 1 ou 2 et

$R^2$ et $R^3$, ayant des significations identiques ou différentes, représentent chacun l'hydrogène, un groupe alkyle en $C_1$-$C_5$ ou alcanoyle en $C_1$-$C_5$, un reste de formule :

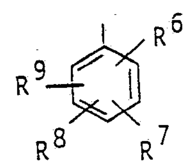

dans laquelle $R^6$, $R^7$, $R^8$ et $R^9$ représentent chacun, indépendamment les uns des autres, l'hydrogène, un halogène, un groupe alkyle contenant jusqu'à 6 atomes de carbone, aryle, un groupe —$OCOR^{10}$, un groupe

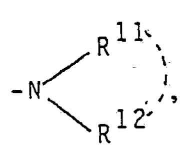

nitro, cyano, alcoxy en $C_1$-$C_4$, alkylthio en $C_1$-$C_4$, hydroxycarbonyle, (alcoxy en $C_1$-$C_4$)-carbonyle, aminocarbonyloxy, sulfonyle ou sulfo, un groupe aryle est un groupe hétérocyclique à 5 ou 6 chaînons, $R^{10}$ représente un groupe alkyle ramifié ou non contenant jusqu'à 6 atomes de carbone, $R^{11}$ et $R^{12}$, ayant des significations identiques ou différentes, représentent chacun l'hydrogène, un groupe alkyle ou alcanoyle contenant chacun jusqu'à 6 atomes de carbone dans la partie alkyle, ou un noyau hétérocyclique à 5 ou 6 chaînons contenant dans le cycle, en tant qu'hétéroatomes, l'azote, l'oxygène ou le soufre, et leurs sels non toxiques acceptables pour l'usage pharmaceutique.

2. Composés selon la revendication 1, dans lesquels n = 2.

3. Composés selon la revendication 2, en configuration Z.

4. Composés selon les revendications 1 à 3, dans lesquels

A représente un groupe alkyle contenant jusqu'à 12 atomes de carbone éventuellement substitué comme indiqué dans la revendication 1, ou un groupe phényle éventuellement substitué comme indiqué dans la revendication 1,

X représente le soufre,

T représente l'hydrogène, un groupe alkyle en $C_1$-$C_4$, un halogène, un groupe alcoxy en $C_1$-$C_4$, hydroxyméthyle, formyloxyméthyle, (alkyle en $C_1$-$C_4$)-carbonyloxyméthyle, aminocarbonyloxyméthyle, pyridiniumméthyle, 4-carbamoylpyridinium-méthyle, halogénométhyle, dichloracétoxyméthyle, sulfonyloxyméthyle ou hétérocyclylthiométhyle.

5. Composés selon les revendications 1 à 4, dans lesquels :

Y a la signification IIb ou IIa,

dans cette dernière,

X représente le soufre,

T représente l'hydrogène, un halogène ou un groupe :

$-CH_2OCOCH_3$ , 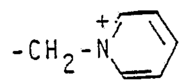 , 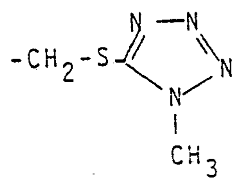 ,

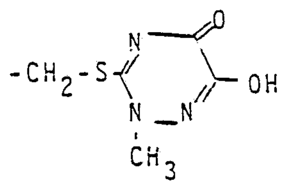 , 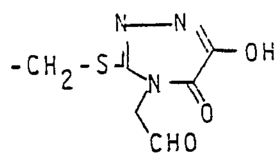 ou

$-CH_2OCONH_2$, et dans lesquels :

n = 2,

Z représente l'hydrogène ou un groupe méthoxy, et

A représente un groupe hydroxy, alkyle contenant jusqu'à 6 atomes de carbone ou un groupe phényle non substitué ou substitué comme indiqué dans la revendication 1.

6. Composés répondant à la formule (III) :

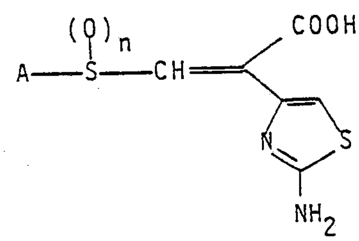

(III)

dans laquelle A et n ont les significations indiquées dans la revendication 1.

7. Composés selon la revendication 6 en configuration Z.

8. Procédé de préparation des composés de formule (I) :

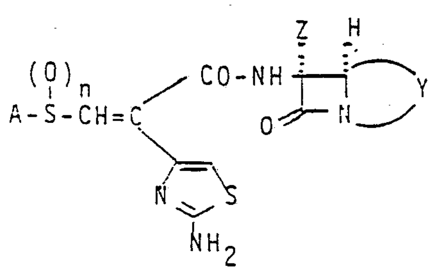

(I)

dans laquelle :

Z représente l'hydrogène ou un groupe alcoxy en $C_1$-$C_6$,

n est égal à 1 ou 2,

Y, à l'état d'acide libre, est un reste de formule IIa ou IIb :

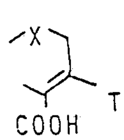 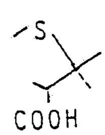

(IIa) (IIb)

dans lesquelles :

X représente —S—, —SO— ou —$SO_2$—, et

T représente un reste usuel dans la chimie des céphalosporines,

et dans lesquels :

A représente un groupe hydroxy, alkyle contenant jusqu'à 18 atomes de carbone, éventuellement substitué par des halogènes, des groupes hydroxy, alcoxy en $C_1$-$C_4$, $OCOR^2$, thio, un groupe

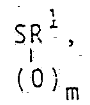

un groupe

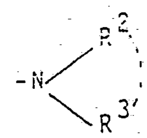

nitro, cyano, hydroxycarbonyle, (alcoxy en $C_1$-$C_4$)-carbonyle, aminocarbonyloxy, sulfo ou un hétérocycle à 5 ou 6 chaînons,

$R^1$ représente un groupe alkyle en $C_1$-$C_4$, ramifié ou non,

m est égal à 0, 1 ou 2, et

$R^2$ et $R^3$, ayant des significations identiques ou différentes, représentent chacun l'hydrogène, un groupe alkyle en $C_1$-$C_5$ ou alcanoyle en $C_1$-$C_5$, un reste de formule :

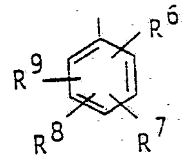

dans laquelle $R^6$, $R^7$, $R^8$ et $R^9$ représentent chacun, indépendamment les uns des autres, l'hydrogène, un halogène, un groupe alkyle contenant jusqu'à 6 atomes de carbone, aryle, un groupe —$OCOR^{10}$, un groupe

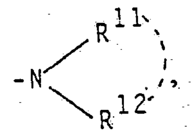

nitro, cyano, alcoxy en $C_1$-$C_4$, alkylthio en $C_1$-$C_4$, hydroxycarbonyle, (alcoxy en $C_1$-$C_4$)-carbonyle, aminocarbonyloxy, sulfonyle ou sulfo, un groupe aryle étant un groupe hétérocyclique à 5 ou 6 chaînons, $R^{10}$ représente un groupe alkyle droit ou ramifié contenant jusqu'à 6 atomes de carbone, $R^{11}$ et $R^{12}$ ayant des significations identiques ou différentes, représentent chacun l'hydrogène, un groupe alkyle ou alcanoyle contenant chacun jusqu'à 6 atomes de carbone dans la partie alkyle, ou un noyau hétérocyclique à 5 ou 6 chaînons, contenant l'azote, l'oxygène ou le soufre en tant qu'hétéroatomes dans le cycle, caractérisé en ce que :

a) on fait réagir des composés de formule (III)

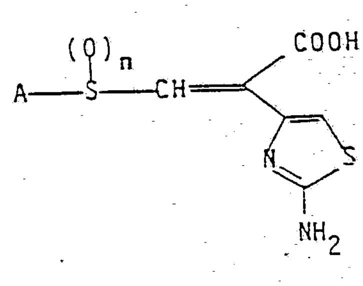

(III)

dans laquelle A et n ont les significations indiquées ci-dessus, de manière connue en soi, avec des composés de formule (IV)

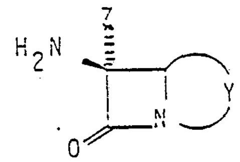

(IV)

dans laquelle X et Y ont les significations indiquées ci-dessus, ou bien,

b) pour obtenir les composés dans lesquels Z = $OCH_3$, on alcoxyle de manière connue en soi les composés de formule (I) dans laquelle Z = H, ou bien

c) on fait réagir les composés de formule (I) dans laquelle Y a la signification IIa, T représente un groupe $CH_2$—O—CO— alkyle inférieur ou halogénométhyle, sulfonyloxyméthyle ou dichloracétoxyméthyle, avec des agents nucléophiles, ou bien

d) on fait réagir des composés de formule (I) dans laquelle Y a la signification IIa et T représente un groupe —$CH_2$—OH, avec O=C=N—$SO_2$—Cl ou O=C=N—$COCl_3$, puis on élimine ensuite le groupe $SO_2Cl$ ou $COCCl_3$.

9. Utilisation des composés de formule générale (I) selon la revendication 8 pour la préparation de médicaments.